# EUROPÄISCHE PATENTSCHRIFT

(11) **EP 1 381 624 B1**
(45) Veröffentlichungstag und Bekanntmachung des Hinweises auf die Patenterteilung: **15.10.2008**
(21) Anmeldenummer: 02735235.0
(22) Anmeldetag: 08.04.2002
(51) Int. Cl.: C07K 14/00, C12Q 1/68, G01N 33/68, B01J 19/00

(54) **SPEZIFISCHER NACHWEIS VON PROTEOLYTISCHEN ENZYMEN MITTELS EINES HYBRID-PROTEINS**
SPECIFIC DETECTION OF PROTEOLYTIC ENZYMES BY MEANS OF A HYBRID PROTEIN
DETECTION SPECIFIQUE D'ENZYMES PROTEOLYTIQUES AU MOYEN D'UN PROTEIN HYBRIDE

(30) Priorität: 06.04.2001 DE 10117430
(43) Veröffentlichungstag der Anmeldung: 21.01.2004
(73) Patentinhaber: Wolfrum, Jürgen, 69120 Heidelberg (DE); Knemeyer, Jens-Peter, 69214 Eppelheim (DE); Sauer, Markus, 68124 Heidelberg (DE); Marmé, Nicole, 69124 Eppelheim (DE)
(72) Erfinder: MARMÉ , Nicole, 69214 Eppelheim (DE)
(74) Vertreter: Köllner, Malte
(86) Internationale Anmeldenummer: PCT/EP2002/003882
(87) Internationale Veröffentlichungsnummer: WO 2002/081509

(56) Entgegenhaltungen:
- EP-A- 0 428 000
- NG M ET AL: "A FLUORESCENT OLIGOPEPTIDE ENERGY TRANSFER ASSAY WITH BROAD APPLICATIONS FOR NEUTRAL PROTEASES" ANALYTICAL BIOCHEMISTRY, Bd. 183, Nr. 1, 1989, Seiten 50-56, XP009001310 ISSN: 0003-2697
- SMITH^A R ET AL: "Evidence for the activation of PAR-2 by the sperm protease, acrosin: expression of the receptor on oocytes" FEBS LETTERS, ELSEVIER SCIENCE PUBLISHERS, AMSTERDAM, NL, Bd. 484, Nr. 3, 10. November 2000 (2000-11-10), Seiten 285-290, XP004337756 ISSN: 0014-5793
- OLSEN MARK J ET AL: "Function-based isolation of novel enzymes from a large library." NATURE BIOTECHNOLOGY, Bd. 18, Nr. 10, Oktober 2000 (2000-10), Seiten 1071-1074, XP002221720 ISSN: 1087-0156

## Beschreibung

Proteolytische Enzyme sind Proteasen oder Peptidasen, also Enzyme, die Peptide bzw. Proteine zerschneiden bzw. lysieren können. Das Wort "Peptid" bezeichnet in der Regel eine kürzere Aminosäuresequenz, während mit dem Wort "Protein" in der Regel größere, aus einer Aminosäuresequenz bestehende Moleküle bezeichnet werden. Beide Begriffe werden im Folgenden synonym verwendet.

Peptidasen und Proteasen spielen eine entscheidende Rolle bei der Proteinaktivierung, Zellregulation und Signalübertragung. Ihr möglichst empfindlicher Nachweis ist daher von enormer Wichtigkeit für das Verständnis der Funktionsweise einer Zelle und deren Kommunikation sowie für die Verlaufskontrolle von Krankheiten.

Des Weiteren erlaubt die genaue Kenntnis der Peptidasen- bzw. Proteasenkonzentration die gezielte Entwicklung neuer Therapeutika zur Inhibierung, z. B. der HIV-Protease. Die HIV-Protease zerlegt das nach der Vermehrung in einer Zelle zunächst sehr lange HIV-Protein in funktionsfähige Einzelproteine, die erst das weitere Wirken und Wachsen des HIV-Virus ermöglichen. Dadurch ergeben sich zwei Ansatzpunkte. Zum einen: Wird die HIV-Protease blockiert, kann sich der HIV-Virus nicht vermehren und seine Ausbreitung wird unterbunden. Zum anderen kann die Ansteckung mit dem HIV-Virus über einen Nachweis der HIV-Protease erfolgen. Der spezifische Nachweis der HIV-Protease kann daher als AIDS-Test dienen.

Auch gewinnen Protease-Tests zunehmend in der medizinischen Forschung an Interesse, da immer mehr Krankheiten, u. a. Krebs, mit Enzymen in Verbindung gebracht werden. Tumorassoziierte Proteasen sind in zunehmendem Maße Gegenstand onkologisch-biochemischer Forschung. Diese Proteasen bewirken den Abbau von Proteinen des Tumorstromas und der Basalmembran und ermöglichen so die Tumorzellinvasion. Deshalb gehören in der Tumordiagnostik Untersuchungen von Protease-Systemen, die in Tumorzellen überexprimiert werden, zu den neuen Prognosefaktoren.

Wichtig ist auch der Einsatz von Protease-Tests in der Qualitätskontrolle biochemischer Produkte. Werden z. B. Proteine, Enzyme, Peptide, etc. verkauft, so muss sichergestellt werden, dass die verkauften Produkte frei von Proteasen sind, damit sich das verkaufte Produkt nicht von allein auflöst. Gleiches kann auch zum Test der spezifischen Aktivität von Proteasen eingesetzt werden, z. B. nach längerer Lagerung, hinsichtlich der Frage, ob die Enzyme noch aktiv sind.

Die proteolytischen Enzyme werden je nach Angriffspunkt im Substrat in Endopeptidasen (Proteasen) und Exopeptidasen unterteilt. Endopeptidasen schneiden Aminobindungen im Inneren von Peptiden. Exopeptidasen verdauen Peptide Aminosäure für Aminosäure von ihren Enden her, d. h. sie schneiden terminale Aminosäuren. Die Exopeptidasen können weiter in Aminopeptidasen und Carboxypeptidasen entsprechend ihrer Aktivität am Amino- oder N-Terminus bzw. am Carboxy- oder C-Terminus des Peptids unterteilt werden.

Zur Konzentrations- oder Aktivitätsbestimmung verschiedener Peptidasen und Proteasen wurden bisher verschiedene fluoreszenzbasierte Tests entwickelt, deren Funktionsprinzipien und Empfindlichkeiten im Folgenden kurz vorgestellt werden.

Eine Möglichkeit, proteolytische Enzyme nachzuweisen bietet der so genannte "EnzChek Protease Assay Kit" des Unternehmens Molecular Probes (Eugene, USA). Dieser Kit verwendet zum Nachweis von Proteasen Caseinderivate, an die sehr viele pH-unempfindliche grün oder rot fluoreszierende BODIPY-Farbstoffe (Karolin J, Johansson BA, Strandberg L, Ny T; J. Am. Chem. Soc. 1994, 116, 7801-7806) gekoppelt sind. Durch den sehr hohen Markierungsgrad des Proteins sind die intermolekularen Abstände zwischen den Fluorophoren sehr klein (typischerweise im Bereich von wenigen Nanometern). Die Farbstoffe löschen sich daher u. a. durch Dimeren-Bildung gegenseitig. Casein ist ein großes Enzym, das viele Bindungsstellen für Proteasen enthält, da es viele unterschiedliche Sequenzabschnitte aufweist. Kommt das farbstoffmarkierte Caseinderivat mit Proteasen in Kontakt, z. B. mit Trypsin, das Peptidbindungen der basischen Aminosäuren Arginin und Lysin auf der Carboxyseite hydrolysiert, so zerschneiden diese das Caseinderivat in kleinere Peptide, wodurch der Abstand zwischen den Farbstoffen in der Regel zunimmt. Dadurch unterbleibt die Löschung und es ist ein Fluoreszenzanstieg zu beobachten, der auf das Vorhandensein von proteolytischen Enzymen hinweist. Die Nachweisempfindlichkeit dieses Kits liegt für Trypsin bspw. bei wenigen ng/ml. Für diese geringen Proteasemengen steigt die Nachweiszeit bzw. Messzeit auf über 10 Stunde. Dieser Test weist viele proteolytische Enzyme unspezifisch nach, denn Casein wird von Elastase, Pepsin, Thermolysin, Papain, Trypsin, etc. verdaut.

Ein auf Rhodamin 110 basierender Enzymtest für verschiedene Proteasen und Peptidasen (Leytus SP, Melhado LL, Mangel WF: Rhodamine-based compounds as fluorogenic substrates for serine protease, (1983) Biochem. J. 209(2):299-307; Leytus SP, Patterson WL, Mangel WF: New class of sensitive and selective fluorogenic substrates for serine proteinases. Amino acid and dipeptide derivatives of rhodamine, (1983) Biochem. J. 215(2):253-260) wird ebenso für den Nachweis von Serinproteasen vermarktet. Hierbei wird mindestens eine der Aminogruppen von Rhodamin 110 kovalent mit einer Aminosäure (meist Arginin) verknüpft, wodurch die Fluoreszenz des Fluorophors stark gelöscht wird. Nach Verdau der Peptidbindungen wird die Fluoreszenz des Rhodamin 110 drastisch erhöht. Die Empfindlichkeit des Tests liegt für Caspase-3 bei wenigen ng Absolutmenge an Enzym. Nachteilig ist, dass das Prinzip nicht allgemein anwendbar ist, teilweise unspezifisch arbeitet und relativ unempfindlich ist.

Auch kann ein Protease- oder Peptidase-Test mit Hilfe des Förster-Fluoreszenz-Resonanz-Energie-Transfers (FRET) (Forster Th: Zwischenmolekulare Energiewanderung und Fluoreszenz, (1948) Annalen der Physik 2:55-75) realisiert werden. Hierbei wird die spezifische Erkennungssequenz einer Endopeptidase an den beiden Enden mit je einem Donorfarbstoff bzw. einem Akzeptorfarbstoff kovalent markiert. Aufgrund der spektralen Überlappung der Donoremission mit der Akzeptorabsorption wird die Fluoreszenz des Donors bei kleinen Abständen gelöscht. Wird das Peptid durch die Endopeptidase geschnitten, geht der räumlich nahe Kontakt zwischen Donor und Akzeptor verloren, es resultiert ein Fluoreszenzanstieg. Als Beispiel sei hier der Nachweis der HIV-Protease kurz erläutert. Hierbei wird die spezifische Schneidesequenz der HIV-Protease jeweils nahe der Schnittstelle mit einem EDANS-Farbstoff (Donor) und einem DABCYL-Farbstoff (Akzeptor) verknüpft. Die PeptidSequenz lautet dann: Arg-Glu(EDANS)-Ser-Gln-Asn-Tyr-Pro-Ile-Val-Gln-Lys (DABCYL)-Arg, wobei der 3-Buchstaben-Abkürzungscode für die Aminosäuren verwendet wurde. Nach dem Schnitt durch die HIV-Protease erhält man zwei Teile: Arg-Glu(EDANS)Ser-Gln-Asn-Tyr-OH und Pro-Ile-Val-Gln-Lys(DABCYL)-Arg. Das Prinzip ist allgemein anwendbar, es muss jedoch ein geeigneter kleiner Abstand zwischen den beiden Farbstoffen gewährleistet sein. Die Empfindlichkeit liegt bei nanomolaren Lösungen von HIV-Protease. Nachteilig ist die aufwendige Synthese des Substrates mit spezifischer Kopplung eines Donors und eines Akzeptors. Wenn das Substrat teilweise nur unvollständig markiert ist, z. B. nur mit einem Donor, resultiert eine schlechte Empfindlichkeit des Tests.

Einen auf FRET basierenden Peptidase-Test, bei dem die Aminosäure Tryptophan als Fluorphor und Dansyl als Quencher eingesetzt werden, beschreiben Ng et al. (Ng M, Auld DS: A Fluorescent Oligopeptide Energy Transfer Assay with Broad Applications for Neutral Proteases, (1989) Anal. Biochem. 183:50-56).

Aufgabe der Erfindung ist es, die Möglichkeiten für einen spezifischen Nachweis von proteolytischen Enzymen zu verbessern.

Diese Aufgabe wird durch die Erfindungen gemäß den unabhängigen Ansprüchen gelöst. Vorteilhafte Weiterbildungen sind in den Unteransprüchen beschrieben.

Ein erstes Nachweisverfahren (kurz "Test") dient zum Nachweis von Endopeptidasen (im Folgenden auch kurz Enzyme) mittels spezieller Peptide.

Dazu wird ein Peptid mit den im Folgenden aufgezählten Eigenschaften präpariert. Das Peptid enthält eine Erkennungssequenz der nachzuweisenden Endopeptidase und kann daher für das Enzym als Substrat fungieren, wobei sich eine Schnittstelle zwischen zwei Aminosäuren bei der Erkennungssequenz im Peptid ergibt.

Das Peptid enthält mindestens einen Quencher und mindestens einen Fluorophor. Mit "Quenchen" wird die Reduktion der Emission des Fluorophors aufgrund der Wechselwirkung mit eben dem Quencher bezeichnet. Als Fluorophor wird ein Fluoreszenzfarbstoff verwendet. Das Quenchen bewirkt eine Verringerung der Fluoreszenzquantenausbeute. Statt "Quenchen" und "Quencher" werden synonym auch die Wörter "Löschen" und "Löschmolekül" bzw. "Löscher" verwendet.

Als Quencher wird die Aminosäure Tryptophan verwendet. Andere Aminosäuren, wie Tyrosin oder Histidin, zeigen auch eine leichte Fluoreszenzlöschung, haben aber nicht die stark löschende Wirkung von Tryptophan.

Der Fluoreszenzfarbstoff wird derart gewählt, dass seine Fluoreszenz von Tryptophan durch photoinduzierten Elektronentransfer gelöscht werden kann, wobei Tryptophan als Elektronen-Donor wirkt. Ferner sollte der Fluoreszenzfarbstoff an Peptide oder Proteine kovalent gekoppelt werden können. Der Fluorophor wird in der Regel an eine Aminosäure gekoppelt.

Um eine hohe Empfindlichkeit des Tests zu erreichen, muss die Sequenz des Peptids gezielt gewählt werden. Und zwar muss der Quencher auf einer Seite der Schnittstelle angeordnet sein und der Fluorophor auf der anderen Seite der Schnittstelle. Die Sequenz muss so gewählt werden, dass eine hinreichende räumliche Nähe zwischen Quencher und Fluorophor gegeben ist, solange das Substrat noch nicht durch das Enzym an der Schnittstelle zwischen Quencher und Fluorophor geschnitten wurde, um die Möglichkeit einer mindestens teilweisen Löschung der Emission des Fluorophors zu gewährleisten. Nach dem Schneiden des Substrats durch das Enzym an der Schnittstelle zwischen Quencher und Fluorophor muss ein deutlicher Anstieg der Emission des Fluorophors möglich sein.

Die Markierung des Peptids bzw. Proteins mit dem Fluoreszenzfarbstoff erfolgt also nicht am Tryptophan selbst, sondern an einer anderen Aminosäure, die durch die Schnittstelle vom Tryptophan getrennt ist. Außerdem wird eine andere Aminosäure als Tryptophan zum Ankoppeln des Fluoreszenzfarbstoffs gewählt, z. B. Lysin, Arginin oder Cystein. Der Fluoreszenzfarbstoff kann auch an ein Ende des Peptids bzw. Proteins gekoppelt werden.

Den größten Anstieg der Fluoreszenz erhält man, wenn sich das Tryptophan und die farbstoffmarkierte Aminosäure unmittelbar zu beiden Seiten der Schnittstelle befinden. Für den Farbstoff MR 121 z. B. kann nach Schneiden des Peptids durch das Enzym ein Anstieg der Fluoreszenz um einen Faktor 10 beobachtet werden.

Ferner darf kein Tryptophan an benachbarten Positionen auf der selben Seite der Schnittstelle der Peptidsequenz angeordnet sein wie der Farbstoff, insbesondere nicht an der unmittelbar benachbarten, damit der Farbstoff nicht durch dieses weitere Tryptophan gequencht wird.

Die geeignete Wahl der Sequenz des Substrats ermöglicht es, das Substrat mit lediglich einem Fluoreszenzfarbstoff zu markieren und nicht mit mindestens zweien, wie es bisher erforderlich war. Dies vereinfacht die Synthese des Substrats. Auch wird die Bindung des Enzyms an das Substrat weniger durch angekoppelte Farbstoffe gestört, wodurch die Spezifität der Bindung zwischen Substrat und Enzym erhöht ist.

Schließlich werden das solcherart präparierte Peptid und das nachzuweisende Enzym in einer Lösung gemischt. Kommt es zu einer Bindung zwischen Peptid und Peptidase, so wird das Peptid an der Schnittstelle geschnitten. Der Fluorophor entfernt sich dadurch räumlich vom Quencher und die Emission des Fluorophors erhöht sich. Dieser Anstieg der Emission wird bestimmt und dient zum Nachweis derjenigen Peptidasen oder Proteasen, die an die Erkennungssequenz binden und dort das Peptid schneiden können. Die Nachweisreaktion ist also spezifisch. Sie kann durch Variation der Peptidsequenz für den Nachweis spezifischer Enzyme ausgerichtet werden.

Insbesondere kann der Test Enzyme spezifisch nachweisen, auch wenn andere Enzymarten in der Lösung mit vorhanden sind. So kann z. B. das Enzym Trypsin in einer Lösung nachgewiesen werden, die auch Elastase und Chymotrypsin enthält.

Wird der zeitliche Verlauf des Anstiegs der Emission verfolgt, kann die Konzentration des Enzyms quantitativ bestimmt werden. Mit Hilfe des erfindungsgemäßen Tests lassen sich auch Proteasen bzw. Peptidasen in einer Konzentration von 10^-15 Mol/l nachweisen.

Als Farbstoffe kommen alle Fluoreszenzfarbstoffe in Betracht, die durch die Aminosäure Tryptophan in ihrer Fluoreszenz messbar gelöscht werden. Vorzugsweise eignen sich Farbstoffe der Klassen der Oxazin-Derivate (z. B. MR 121, JA 242, JA 243) und Rhodamine (z. B. JA 165, JA 167, JA 169), die im roten Spektralbereich des sichtbaren Lichts absorbieren und emittieren [siehe z. B. Müller R, Zander C, Sauer M, Deimel M, Ko DS, Siebert S, Arden-Jacob J, Deltau G, Marx NJ, Drexhage KH, Wolfrum J. Chem. Phys. Let. 1996, 262, 716-722]. Diese roten Farbstoffe werden um ein bis zwei Größenordnungen besser durch Tryptophan gelöscht, als sonstige Farbstoffe.

Durch den Einsatz von roten Farbstoffen wird die Empfindlichkeit deutlich erhöht, da andernfalls die meistens im grünen Spektralbereich des sichtbaren Lichts liegende Autofluoreszenz biologisch relevanter Proben, wie beispielsweise Blutseren und Ascites, einen empfindlichen Nachweis erschwert. Mit dem erfindungsgemäßen Verfahren können auch in diesen biologisch relevanten Proben empfindliche Test durchgeführt werden, sogar in unverdünnten Blutseren.

Die Aufgabe wird auch durch einen zweiten Test zum Nachweis von Exopeptidasen (Enzym) gelöst.

Wiederum wird ein Peptid präpariert. Diesmal enthält das Peptid mindestens eine Gruppe, die einen Quencher und ein Fluorophor an unterschiedlichen Aminosäuren enthält. Als Quencher wird wiederum die Aminosäure Tryptophan verwendet. Als Fluorophor wird wiederum ein Fluoreszenzfarbstoff verwendet. Der Fluoreszenzfarbstoff wird derart gewählt, dass er an Peptide oder Proteine kovalent gekoppelt werden kann. Ferner kann die Fluoreszenz des Farbstoffs durch Tryptophan gelöscht werden.

Die Sequenz des Peptids wird derart gewählt, dass eine hinreichende räumliche Nähe zwischen Quencher und Fluorophor gegeben ist, solange die den Quencher bzw. den Fluoreszenzfarbstoff tragenden Aminosäuren noch nicht durch das Enzym getrennt wurden, um die Möglichkeit einer mindestens teilweisen Löschung der Emission des Fluorophors zu gewährleisten. Ferner muss nach Trennen der den Quencher bzw. den Fluoreszenzfarbstoff tragenden Aminosäuren durch das Enzym eine hinreichende räumliche Entfernung zwischen einem ggf. anderen Quencher und dem Fluorophor gewährleistet sein, um eine mindestens teilweise Erhöhung der Emission des Fluorophors zu erreichen.

Das solcherart präparierte Peptid wird mit dem nachzuweisenden Enzym in einer Lösung gemischt. Die Stärke der Emission des Fluorophors in der Lösung wird bestimmt.

In vielen Aspekten entspricht dieser Test dem bereits geschilderten zum Nachweis einer Endopeptidase. Allerdings ist der Nachweis einer Exopeptidase leichter.

Die Exopeptidase verdaut das Protein oder Peptid von seinem Ende her. Dabei trennt sie Aminosäure für Aminosäure ab. Unter anderem wird auch ein Tryptophan abgetrennt. Der in der Nachbarschaft an eine Aminosäure gekoppelte Farbstoff wird daher nicht mehr gelöscht und es kommt zu einem Fluoreszenzanstieg. Gleiches gilt, wenn die Aminosäure mit dem Farbstoff zuerst abgetrennt wird.

Enthält das Substrat eine Mehrzahl von Tryptophan-Farbstoff-Gruppen, so kann mehrmals ein Fluoreszenzanstieg beobachtet werden. Im Effekt kommt es dann zu einem mehrfachen Fluoreszenzanstieg.

Auch hier können wieder die erwähnten roten Farbstoffe vorteilhaft eingesetzt werden.

Die Aufgabe wird schließlich durch einen dritten Test zum Nachweis von Endopeptidasen (Enzym) gelöst. Dieser Test hat wiederum viele Ähnlichkeiten und einige entscheidende Unterschiede.

Zunächst wird ein Peptid präpariert. An einem Ende des Peptids ist eine Gruppe angeordnet, die es verhindert, dass eine Exopeptidase das Peptid verdaut. Die Gruppe am Ende des Peptids, die das Verdauen des Peptids durch eine Exopeptidase verhindert, wird auch als Stoppgruppe oder Stoppmolekül bezeichnet. Es kann sich dabei beispielsweise um D-Aminosäuren, DNA oder PNA handeln, die von der Exopeptidase nicht geschnitten werden.

Eine weitere Möglichkeit, eine Stoppgruppe zu realisieren, besteht darin, durch geeignete Wahl der Sequenz des Peptids am Ende des Peptids eine Aminosäure oder Aminosäuresequenz anzuordnen, die von der Exopeptidase nicht geschnitten wird. Die Carboxypeptidase A als Exopeptidase, z. B., schneidet nicht über die Aminosäure Prolin am Ende eines Peptids hinweg. Die Anordnung einer gewöhnlichen Aminosäure als Stoppgruppe erleichtert die Synthese des Peptids.

Die Exopeptidase kann sich also mit dem Peptid oder Protein in Lösung befinden, ohne dass dieses angegriffen wird.

Das Peptid enthält ferner eine Erkennungssequenz der nachzuweisenden Endopeptidase und kann daher für das Enzym als Substrat fungieren, wobei sich eine Schnittstelle zwischen zwei Aminosäuren bei der Erkennungssequenz im Peptid ergibt.

Das Peptid enthält von der Stoppgruppe aus betrachtet in der Sequenz des Peptids jenseits der Schnittstelle mindestens eine Gruppe, die einen Quencher und ein Fluorophor an unterschiedlichen Aminosäuren enthält.

Die Sequenz des Peptids wird derart gewählt, dass eine hinreichende räumliche Nähe zwischen Quencher und Fluorophor gegeben ist, solange die den Quencher bzw. den Fluoreszenzfarbstoff tragenden Aminosäuren noch nicht durch eine Exopeptidase getrennt wurden, um die Möglichkeit einer mindestens teilweisen Löschung der Emission des Fluorophors zu gewährleisten.

Außerdem muss gewährleistet sein, dass nach Trennen der den Quencher bzw. den Fluoreszenzfarbstoff tragenden Aminosäuren durch eine Exopeptidase eine hinreichende räumliche Entfernung zwischen einem ggf. anderen Quencher und dem Fluorophor gewährleistet ist, um eine mindestens teilweise Erhöhung der Emission des Fluorophors zu erreichen.

Schließlich werden das solcherart präparierte Peptid, die mindestens eine Exopeptidase und die nachzuweisende Endopeptidase in einer Lösung gemischt. Dadurch kommt es zu einer Bindung zwischen Peptid als Substrat und Endopeptidase als Enzym. Das Peptid wird an der Schnittstelle innerhalb des Moleküls geschnitten. Dadurch wird die schützende Stoppgruppe am Ende des Substrats vom Rest des Substrats getrennt. Der übrig bleibende Rest weist dann keine schützende Stoppgruppe mehr auf, und eine Exopeptidase kann den Rest des Substrats verdauen.

Eine solche Exopeptidase kann der Lösung in ausreichender Menge zugegeben werden, zusammen mit dem Substrat. Sie kann also als Kit zusammen mit dem Substrat eingesetzt werden.

Durch den Verdau mittels der Exopeptidase werden Quencher und Fluorophor getrennt. Es kommt wiederum zu einem Fluoreszenzanstieg.

Zwischen der Stoppgruppe und dem Bereich des Peptids oder Proteins, dass die Quencher-und-Fluorophor-Gruppen trägt, können beliebig lange Aminosäure-Sequenzen liegen, ohne dass die Nachweisempfindlichkeit beeinträchtigt wird. Somit lassen sich auch Enzyme nachweisen, die eine lange Erkennungssequenz besitzen, wie etwa die HIV-Protease.

Wird als Exopeptidase eine Carboxypeptidase eingesetzt, so wird die Stoppgruppe an das C-terminale Ende des Peptids gelegt, damit der anschließende Verdau durch die Carboxypeptidase erfolgen kann. Wird hingegen als Exopeptidase eine Aminopeptidase eingesetzt, so wird die Stoppgruppe an das N-terminale Ende des Peptids gelegt.

Enthält das Substrat eine Mehrzahl von Quencher-und-Fluorophor-Gruppen, so kann wiederum ein mehrfacher Fluoreszenzanstieg beobachtet werden. Dadurch kann die Messdauer erheblich verkürzt werden. Gleichzeitig wird das Signal deutlich erhöht. Der große Vorteil einer mehrfachen Markierung liegt darin, dass durch einen einzigen Schnitt und somit durch eine einzige nachzuweisende Endopeptidase nicht nur ein Fluorophor aktiviert wird, sondern viele. Man erhält dadurch einen deutlicheren Anstieg des Signals in wesentlich kürzerer Zeit. Dadurch wird der Signalanstieg auch für weniger empfindliche Geräte messbar. Auch die Nachweisgrenze verschiebt sich dadurch zu niedrigeren Werten.

Auch dieser Test weist die Endopeptidasen spezifisch nach mit den weiteren Vorteilen, die bereits erwähnt wurden.

Als Fluorophor können verschiedene Farbstoffe gewählt werden, die an eine Aminosäure kovalent gebunden werden. Ein Quencher kann ebenfalls kovalent an eine Aminosäure gebunden werden. Als Kombination bietet sich hierzu z. B. der Quencher DABCYL oder Guanosin und als Fluorophor z. B. die Farbstoffe Fluorescein, Cumarin oder Eosine an.

Auch für diesen dritten Test kann wiederum als Quencher die Aminosäure Tryptophan verwendet werden, zusammen mit einem Fluoreszenzfarbstoff als Fluorophor. Der Fluoreszenzfarbstoff muss an Peptide oder Proteine kovalent koppelbar sein, und seine Fluoreszenz durch Tryptophan gelöscht werden können. Die Vorteile dieser Wahl wurden bereits erläutert.

Auch können wiederum rote Farbstoffe eingesetzt werden.

U. a. für diesen dritten Test gibt es noch weitere Möglichkeiten, das Paar aus Quencher und Fluorophor zu realisieren. Der Quencher kann mindestens ein erster Fluoreszenzfarbstoff sein und der Fluorophor mindestens ein zweiter, sofern beide an Peptide oder Proteine kovalent koppelbar sind. Wichtig ist, dass sich beide Farbstoffe bei räumlicher Nähe gegenseitig löschen können. Dies kann durch Dimeren-Bildung oder andere Mechanismen, etwa Energietransfer, der Fall sein. Nach Entfernung der Stoppgruppe durch die nachzuweisende Endopeptidase verdaut die Exopeptidase die restliche Peptidkette, so dass sich die Farbstoffe voneinander entfernen und somit ein Quenchen nicht mehr möglich ist. Der daraus resultierende Anstieg der Fluoreszenzintensität wird vermessen.

Als Farbstoffe dafür eignen sich hierfür vorzugsweise alle oben aufgeführten Rhodamine und Oxazine. Dazu Cyanin-Derivate (z. B. Cy 3, Cy 5, Cy 7, erhältlich bei Amersham Biosciences), alle BODIPY-Farbstoffe, alle Cumarine, Fluorescein und Texas-Red.

Im Folgenden wird die Erfindung anhand von Ausführungsbeispielen näher erläutert, die in den Figuren schematisch dargestellt sind. Gleiche Bezugsziffern in den einzelnen Figuren bezeichnen dabei gleiche Elemente. Im Einzelnen zeigt:
- Fig. 1: eine schematische Darstellung des Grundprinzips der Nachweisreaktion;
- Fig. 2: eine schematische Darstellung der Abläufe beim photoinduzierten Elektronentransfer;
- Fig. 3: eine schematische Darstellung des Grundprinzips einer Variante der Nachweisreaktion;
- Fig. 4: eine schematische Darstellung der Nachweisreaktion gemäß Fig. 4 mit Mehrfachmarkierung; und
- Fig. 5: die Strukturformeln der zitierten Farbstoffe.

### Erster Test

### Nachweis einer Endopeptidase

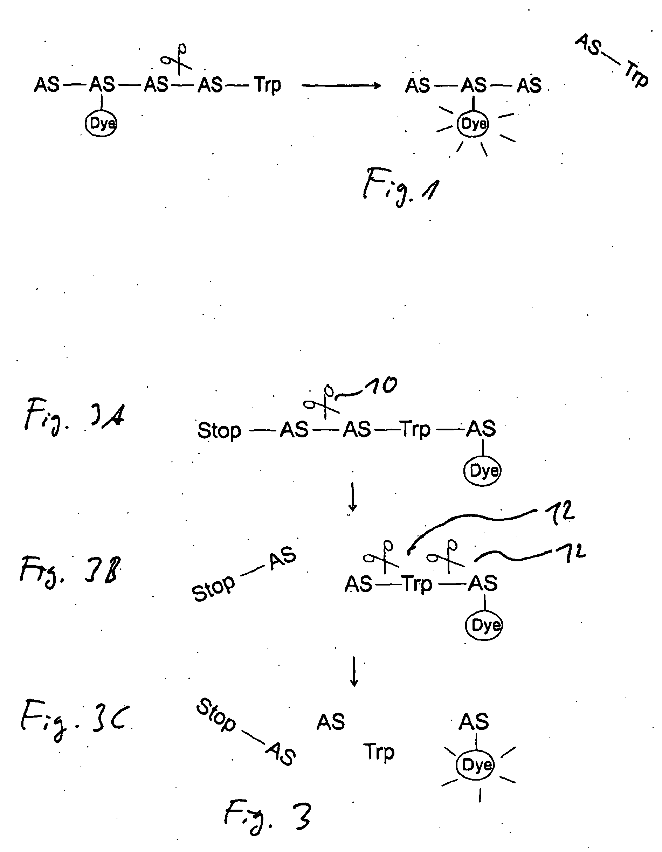

Fig. 1 zeigt schematisch das Prinzip des Nachweises einer Endopeptidase, die in Fig. 1 durch das Scheren-Symbol dargestellt ist. Als Substrat dient das Peptid bestehend aus vier Aminosäuren (AS) und Tryptophan (Trp).

An die zweite Aminosäure von links ist ein Farbstoffmolekül (Dye) gekoppelt, vorzugsweise MR 121. Möglichkeiten der Kopplung sind beschrieben in [Bodanszky M: "Peptide Chemistry: A Practical Textbook", Springer Verlag 1986] [Anderson GW, Zimmerman JE, Callahlan FM: "The use of esters of N-Hydroxysuccinimide in Peptide Synthesis", J. Am. Chem. Soc. 1964, 86, 1839-1842].

Möchte man z. B. die Endopeptidase Trypsin nachweisen, die die Peptidbindung zwischen zwei Arginin hydrolysiert, so kann das folgende Peptid als Substrat synthetisiert werden: Gln-Lys(MR 121)-Arg-Arg-Trp, wie es schematisch in Fig. 1 gezeigt ist.

Das Tryptophan wirkt als Quencher auf den Farbstoff. Der Mechanismus der Löschung ist eine statische Löschung im Gegensatz zu einer dynamischen Stoßlöschung. Der Farbstoff bildet mit Tryptophan einen Komplex, einen sog. Grundzustandskomplex, der kaum fluoresziert. In diesem Grundzustandskomplex kommt es zu einem Elektrontransfer vom Tryptophan auf das angeregte Farbstoffmolekül.

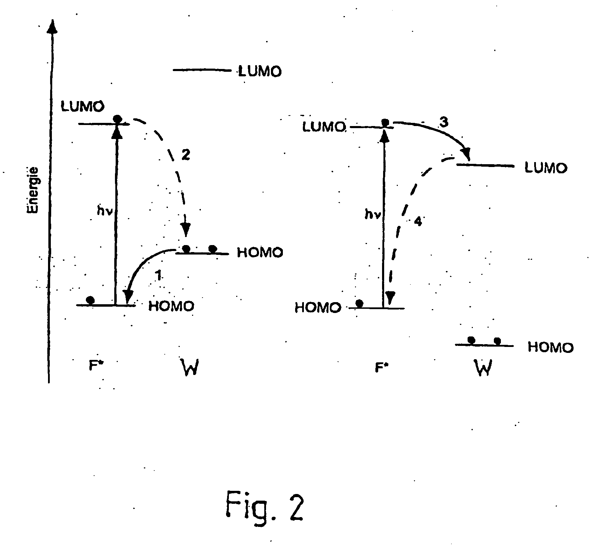

Im Folgenden sei der Elektronentransfer anhand von Fig. 2 kurz erläutert. Dargestellt ist die Fluoreszenzlöschung des angeregten Farbstoff-Moleküls F* durch einen Tryptophan-Rest W. Die schwarzen Kreise repräsentieren Elektronen. Es sind jeweils das HOMO (highest occupied molecular orbital) und das LUMO (lowest unoccupied molecular orbital) eingezeichnet. Das HOMO ist das energetisch höchste, im elektronischen Grundzustand besetzte Molekülorbital. Das LUMO ist das energetisch niedrigste, im elektronischen Grundzustand unbesetzte Molekülorbital; es ist i. d. R. das Molekülorbital, das im ersten angeregten Zustand besetzt wird.

Prinzipiell gibt es zwei Möglichkeiten der Fluoreszenzlöschung durch photoinduzierten Elektronentransfer. Im in Fig. 2 links dargestellten Fall wirkt der Tryptophan-Rest W als Elektronenspender (Donor). Nach Anregung des Fluorophors F* geht ein Elektron vom doppelt besetzten HOMO des Tryptophan-Rests W zum nun einfach besetzen HOMO des Fluorophors F* über (Weg 1). Es kommt zu einer Reduktion des angeregten Fluorophors F* durch den Tryptophan-Rest W. Das Elektron im LUMO des Fluorophors F* kann anschließend zum nun einfach besetzten HOMO des Tryptophan-Rests W übergehen (Weg 2). Dieser Fall tritt zwischen roten Farbstoffen wie MR 121 und Tryptophan auf.

Im in Fig. 2 rechts dargestellten Fall wirkt der Tryptophan-Rest W als Elektronenakzeptor (Akzeptor). Aus dem einfach besetzten LUMO des angeregten Fluorophors F* geht das dort befindliche Elektron zum unbesetzten LUMO des Tryptophan-Rests W über (Weg 3). Es kommt zu einer Oxidation des angeregten Fluorophors F* durch den Tryptophan-Rest W. Das Elektron im LUMO des Tryptophan-Rests W kann anschließend zum HOMO des Fluorophors zurückkehren (Weg 4).

In beiden Fällen kann das Elektron nach dem Elektronentransfer nicht mehr aus dem LUMO des angeregten Fluorophors F* durch Aussenden eines Photons in das HOMO zurückkehren. Der erste angeregte Zustand wurde strahlungslos deaktiviert. Die Fluoreszenz ist gelöscht.

Bei den betrachteten roten Farbstoffen wirkt Tryptophan stets als Elektronen-Donor. Diese Kenntnis ermöglicht z. B. die Wahl von geeigneten Farbstoffen ausgehend von ihren elektrochemisch bestimmten Potentialen.

Die Detektion der Fluoreszenzsignale erfolgt vorzugsweise in Lösung in einer Küvette in konventionellen Fluoreszenzspektrometern. Dies wird im Allgemeinen als "homogener Assay" bezeichnet. Empfindlichere Messungen können mittels konfokaler Spektroskopie durchgeführt werden.

An Stelle der Fluoreszenzintensität kann auch die Fluoreszenzlebensdauer zum Nachweis herangezogen werden. Die Fluoreszenzlebensdauer ist im gelöschten Zustand kürzer als im freien, ungelöschten Zustand.

Eine weitere Möglichkeit des Nachweises eines gelöschten bzw. ungelöschten Zustands bietet die Messung von Polarisationsänderungen. Auch die Polarisationseigenschaften von Emissionen, z. B. Fluoreszenzsignalen, können sich zwischen einem gelöschten und einem ungelöschten Zustand ändern.

Beim zweiten und dritten Test können auch FRET-Systeme zum Einsatz kommen, also Farbstoffpaare, die sich durch Energieübertrag löschen. Diese zeigen häufig unterschiedliche Wellenlängen im gelöschten bzw. ungelöschten Zustand, die zum Nachweis herangezogen werden können.

Als Anregungslichtquellen kommen vorzugsweise Diodenlaser zum Einsatz, ansonsten alle anderen Laser oder Lampen mit passender Wellenlänge.

Für die Bestimmung der Abklingzeit der Fluorophore werden vorzugsweise gepulste Diodenlaser eingesetzt.

Neben dem so genannten homogenen Assay gibt es auch einen so genannten heterogenen Assay. Damit wird im Allgemeinen die Detektion auf einer Oberfläche bezeichnet. Die farbstoffmarkierten Peptide oder Proteine können C-Terminal, N-terminal oder über Aminosäure-Reste (z. B. Cystein oder Lysin) kovalent an eine Oberfläche gebunden werden. Als Oberflächen kommen beispielsweise modifizierte Glasoberflächen (also Chips oder Biochips) oder (ggf. magnetische) Beats in Frage. Dazu können die Oberflächen z. B. mit linearen und/oder vernetzten Polyethylenglykolen oder Hyaluronsäure beschichtet werden, um die Adsorption der Moleküle zu verhindern.

Liegt eine Aminosäure, durch die das Peptid an die Oberfläche gebunden ist, auf der gleichen Seite der Schnittstelle, wie der Farbstoff, so führt ein Schnitt durch das nachzuweisende Enzym dazu, dass der Farbstoff kovalent gebunden auf der Oberfläche zurück bleibt und dort nicht mehr in seiner Fluoreszenz gelöscht wird. Die Fluoreszenz kann dann evaneszent oder mittels Oberflächenscreenings detektiert werden.

Es ist auch möglich, das Peptid derart an der Oberfläche zu immobilisieren, dass das Enzym durch den Schnitt gerade die Verbindung zwischen Farbstoff und Oberfläche löst. Die Fluoreszenz des ungelöschten Farbstoffs kann dann in der Lösung nachgewiesen werden.

Zweiter Test

### Nachweis einer Exopeptidase

Der eingangs geschilderte zweite Test zum Nachweis einer Exopeptidase erfolgt in ganz analoger Weise wie der erste Test. Die Unterschiede wurde eingangs geschildert.

### Dritter Test

### Nachweis einer Endopeptidase

Fig. 3 zeigt schematisch das Prinzip des Nachweises einer Endopeptidase gemäß dem dritten Test. Die nachzuweisende Endopeptidase ist in Fig. 3A durch das Scheren-Symbol 10 dargestellt. Als Substrat dient das Peptid bestehend aus einer Stoppgruppe (Stop), zwei Aminosäuren (AS), Tryptophan (Trp) und einer weiteren Aminosäure (AS), an der ein Farbstoffmolekül (Dye) gekoppelt ist, vorzugsweise MR 121.

Die nachzuweisende Endopeptidase 10 schneidet das Peptid zwischen den beiden benachbarten Aminosäuren AS. Diese spaltet die Stoppgruppe (Stop) zusammen mit einer Aminosäure AS ab (siehe Fig. 3B). Dies ermöglicht es der ebenfalls in der Lösung vorhandenen Exopeptidase 12, die bisher durch die Stoppgruppe (Stop) am Verdau gehindert wurde, das Restpeptid zu verdauen.

Dabei spaltet die Exopeptidase 12 sukzessive zunächst eine Aminosäure AS und dann Tryptophan (Trp) ab. Dies hat zur Folge, dass der Farbstoff (Dye) an der verbliebenen Aminosäure (AS) nicht mehr in räumlicher Nähe zum Tryptophan sich befindet, da das Tryptophan in der Lösung weg diffundiert. Der Farbstoff wird daher nicht mehr durch Tryptophan gelöscht. Seine Fluoreszenz kann als Signalanstieg im Spektrometer nachgewiesen werden.

Zum Nachweis der HIV-Protease kann folgendes Peptid verwendet werden: (N-Terminus) Lys(MR 121)-Trp-Ser-Gln-Asn-Tyr-Pro-Ile-Val-Gln-Pro-Pro (C-Terminus). Dieses Peptid enthält die Erkennungssequenz für den Schnitt durch die HIV-Protease. Dieses Peptid kann in Kombination mit der Carboxypeptidase A verwendet werden, die nicht über Prolin hinweg schneidet. Daher dienen die beiden Prolin-Rest am C-Terminus des obigen Peptids als Stoppgruppe. Die HIV-Protease schneidet zwischen Tyrosin und Prolin. Es ergeben sich zwei Fragmente: Lys(MR 121)-Trp-Ser-Gln-Asn-Tyr-OH und Pro-Ile-Val-Gln-Pro-Pro. Die Stoppgruppe ist damit entfernt und die Carboxypeptidase A kann das erste Fragment vom C-Terminus aus verdauen. Dabei werden auch das farbstoffmarkierte Lysin und Tryptophan getrennt. Ein Fluoreszenzanstieg kann nachgewiesen werden.

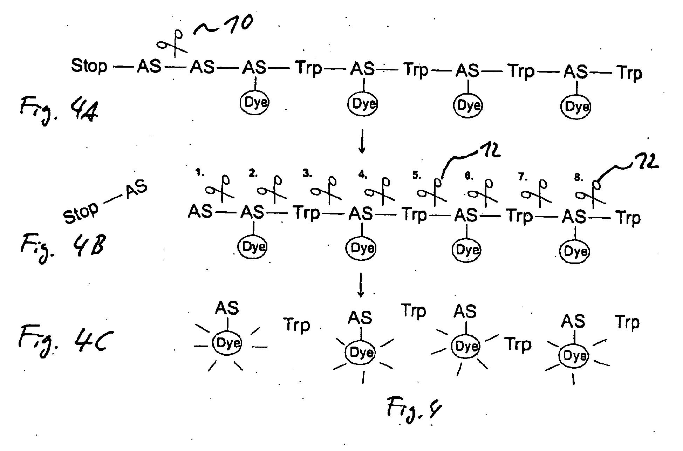

Fig. 4 zeigt den dritten Test bei Signalverstärkung durch Mehrfachmarkierung.

In Fig. 4A ist das Peptid gezeigt ähnlich wie in Fig. 3A, jedoch mit einer Abfolge von abwechselnd Tryptophan (Trp) und einer farbstoffmarkierten Aminosäure (AS + Dye). Spaltet die nachzuweisende Endopeptidase 10 die Stoppgruppe (Stop) ab (siehe Fig. 4B), so kann die Exopeptidase 12 sukzessive das Peptid verdauen. Dabei werden sukzessive (siehe Fig. 4C) Tryptophan und freie Aminosäure mit dem gebundenen Farbstoff (AS + Dye) freigesetzt. Es werden somit durch einen Schnitt der Endopeptidase eine Vielzahl von Farbstoffen freigesetzt, deren Emission nicht mehr gelöscht wird. Es kommt also zu einem starken und schnellen Signalanstieg. Die Messdauer verkürzt sich dadurch auch bei hochempfindlichen Messungen auf Minuten bis Sekunden.

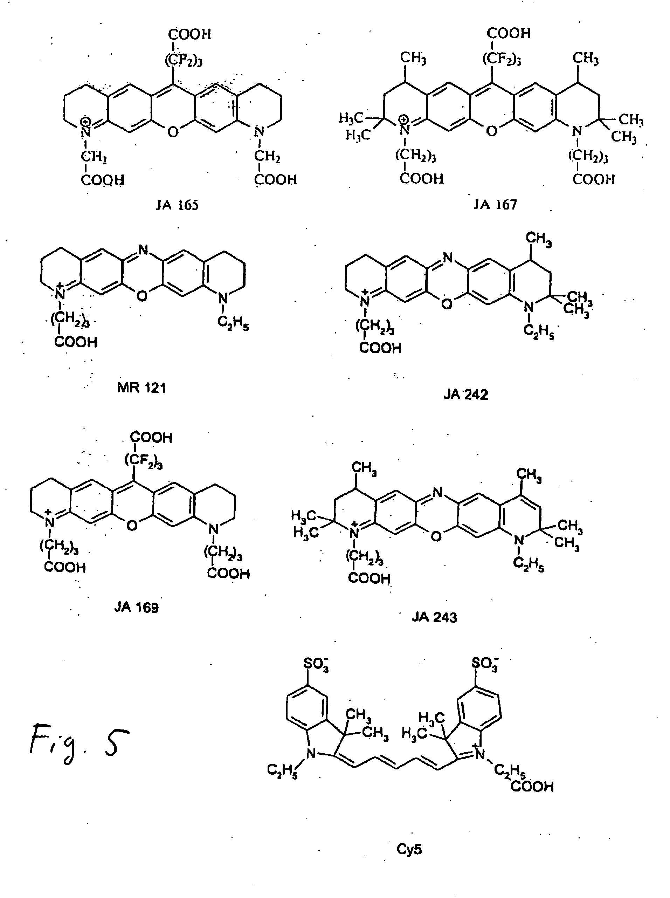

Fig. 5 schließlich zeigt die Struktur der bevorzugt eingesetzten Farbstoffe, wie sie eingangs erwähnt wurden.

## Patentansprüche

1. Peptid mit den folgenden Eigenschaften:
a) das Peptid enthält eine Erkennungssequenz einer Endopeptidase (Enzym) und kann daher für das Enzym als Substrat fungieren, wobei sich eine Schnittstelle zwischen zwei Aminosäuren bei der Erkennungssequenz im Peptid ergibt;
b) das Peptid enthält mindestens einen Quencher und mindestens einen Fluorophor;
c) der Quencher ist die Aminosäure Tryptophan;
d) der Fluorophor ist ein Fluoreszenzfarbstoff;
e) der Fluoreszenzfarbstoff ist an Peptide oder Proteine kovalent koppelbar;
f) die Fluoreszenz des Fluoreszenzfarbstoffs kann von Tryptophan durch photoinduzierten Elektronentransfer gelöscht werden, wobei Tryptophan als Elektronen-Donor wirkt;
g) die Sequenz des Peptids ist wie folgt ausgebildet:
h) der Quencher ist auf einer Seite der Schnittstelle angeordnet;
i) der Fluorophor ist auf der anderen Seite der Schnittstelle angeordnet;
j) eine hinreichende räumliche Nähe zwischen Quencher und Fluorophor ist gegeben, solange das Substrat noch nicht durch das Enzym an der Schnittstelle zwischen Quencher und Fluorophor geschnitten wurde, um die Möglichkeit einer mindestens teilweisen Löschung der Emission des Fluorophors zu gewährleisten;
k) nach dem Schneiden des Substrats durch das Enzym an der Schnittstelle zwischen Quencher und Fluorophor ist ein deutlicher Anstieg der Emission des Fluorophors möglich.

2. Peptid nach dem vorhergehenden Anspruch,
**dadurch gekennzeichnet,**
**dass** der Fluoreszenzfarbstoff ein Oxazin-Derivat oder ein Rhodamin-Derivat ist.

3. Verfahren zum Nachweis einer Endopeptidase (Enzym) mit folgenden Schritten:
mische ein Peptid nach einem der vorhergehenden Ansprüche und das nachzuweisende Enzym in einer Lösung; und
bestimme die Stärke der Emission des Fluorophors in der Lösung.

4. Vorrichtung, auf der ein Peptid nach Anspruch 1 oder 2 immobilisiert ist.

5. Vorrichtung nach dem vorhergehenden Anspruch,
**dadurch gekennzeichnet,**
**dass** das Peptid derart auf der Vorrichtung immobilisiert ist, dass
der Fluorophor nach einem Schnitt durch das nachzuweisende Enzym auf der Oberfläche immobilisiert bleibt, während der Quencher nicht mehr auf der Oberfläche immobilisiert ist.

6. Peptid mit den folgenden Eigenschaften:
a) das Peptid enthält mindestens eine Gruppe, die einen Quencher und ein Fluorophor an unterschiedlichen Aminosäuren enthält;
b) der Quencher ist die Aminosäure Tryptophan;
c) der Fluorophor ist ein Fluoreszenzfarbstoff;
d) der Fluoreszenzfarbstoff ist an Peptide oder Proteine kovalent koppelbar;
e) die Fluoreszenz des Fluoreszenzfarbstoffs kann durch Tryptophan gelöscht werden;
f) die Sequenz des Peptids ist wie folgt ausgebildet:
g) eine hinreichende räumliche Nähe zwischen Quencher und Fluorophor ist gegeben, solange die den Quencher bzw. den Fluoreszenzfarbstoff tragenden Aminosäuren noch nicht durch eine Exopeptidase (Enzym) getrennt wurden, um die Möglichkeit einer mindestens teilweisen Löschung der Emission des Fluorophors zu gewährleisten;
l) nach Trennen der den Quencher bzw. den Fluoreszenzfarbstoff tragenden Aminosäuren durch das Enzym ist eine hinreichende räumliche Entfernung zwischen einem ggf. anderen Quencher und dem Fluorophor gewährleistet, um eine mindestens teilweise Erhöhung der Emission des Fluorophors zu gewährleisten.

7. Peptid nach dem vorhergehenden Anspruch,
**dadurch gekennzeichnet,**
**dass** der Fluoreszenzfarbstoff ein Oxazin-Derivat oder ein Rhodamin-Derivat ist.

8. Peptid nach einem der beiden vorhergehenden Ansprüche,
**dadurch gekennzeichnet,**
**dass** der Quencher mindestens ein erster Fluoreszenzfarbstoff ist; dass der Fluorophor mindestens ein zweiter Fluoreszenzfarbstoff ist;
**dass** der mindestens eine erste und zweite Fluoreszenzfarbstoff an Peptide oder Proteine kovalent koppelbar ist; und
**dass** sich der mindestens eine erste und zweite Fluoreszenzfarbstoff gegenseitig löschen können.

9. Verfahren zum Nachweis einer Exopeptidase (Enzym) mit folgenden Schritten:
mische ein Peptid nach einem der drei vorhergehenden Ansprüche und das nachzuweisende Enzym in einer Lösung; und
bestimme die Stärke der Emission des Fluorophors in der Lösung.

10. Vorrichtung, auf der ein Peptid nach einem der Ansprüche 6 bis 8 immobilisiert ist.

11. Vorrichtung nach dem vorhergehenden Anspruch,
**dadurch gekennzeichnet,**
**dass** das Peptid derart auf der Vorrichtung immobilisiert ist, dass
der Fluorophor nach einem Schnitt durch das nachzuweisende Enzym auf der Oberfläche immobilisiert bleibt, während der Quencher nicht mehr auf der Oberfläche immobilisiert ist.

12. Peptid mit den folgenden Eigenschaften:
a) an einem Ende des Peptids ist eine Gruppe angeordnet, die es verhindert, dass eine Exopeptidase das Peptid verdaut (Stoppgruppe);
b) das Peptid enthält eine Erkennungssequenz einer Endopeptidase (Enzym) und kann daher für das Enzym als Substrat fungieren, wobei sich eine Schnittstelle zwischen zwei Aminosäuren bei der Erkennungssequenz im Peptid ergibt;
c) das Peptid enthält von der Stoppgruppe aus betrachtet in der Sequenz des Peptids jenseits der Schnittstelle mindestens eine Gruppe, die einen Quencher und ein Fluorophor an unterschiedlichen Aminosäuren enthält;
d) die Sequenz des Peptids ist wie folgt ausgebildet:
e) eine hinreichende räumliche Nähe zwischen Quencher und Fluorophor ist gegeben, solange die den Quencher bzw. den Fluoreszenzfarbstoff tragenden Aminosäuren noch nicht durch eine Exopeptidase getrennt wurden, um die Möglichkeit einer mindestens teilweisen Löschung der Emission des Fluorophors zu gewährleisten;
f) nach Trennen der den Quencher bzw. den Fluoreszenzfarbstoff tragenden Aminosäuren durch eine Exopeptidase ist eine hinreichende räumliche Entfernung zwischen einem ggf. anderen Quencher und dem Fluorophor gewährleistet, um eine mindestens teilweise Erhöhung der Emission des Fluorophors zu gewährleisten.

13. Peptid nach dem vorhergehenden Anspruch,
**dadurch gekennzeichnet,**
**dass** der Quencher die Aminosäure Tryptophan ist;
**dass** der Fluorophor ein Fluoreszenzfarbstoff ist;
**dass** der Fluoreszenzfarbstoff an Peptide oder Proteine kovalent koppelbar ist; und
**dass** die Fluoreszenz des Fluoreszenzfarbstoffs durch Tryptophan gelöscht werden kann.

14. Peptid nach dem vorhergehenden Anspruch,
**dadurch gekennzeichnet,**
**dass** der Fluoreszenzfarbstoff ein Oxazin-Derivat oder ein Rhodamin-Derivat ist.

15. Peptid nach Anspruch 12,
**dadurch gekennzeichnet,**
**dass** der Quencher mindestens ein erster Fluoreszenzfarbstoff ist; dass der Fluorophor mindestens ein zweiter Fluoreszenzfarbstoff ist;
**dass** der mindestens eine erste und zweite Fluoreszenzfarbstoff an Peptide oder Proteine kovalent koppelbar ist; und
**dass** sich der mindestens eine erste und zweite Fluoreszenzfarbstoff gegenseitig löschen können.

16. Peptid nach dem vorhergehenden Anspruch,
**dadurch gekennzeichnet,**
**dass** als Fluoreszenzfarbstoff ein Oxazin-Derivat oder ein Rhodamin-Derivat oder eine Cyanin-Derivat ist.

17. Kit
mit einem Peptid nach einem der fünf vorhergehenden Ansprüche und mit mindestens einer Exopeptidase.

18. Verfahren zum Nachweis einer Endopeptidase mit folgenden Schritten:
mische ein Peptid nach einem der Ansprüche 12 bis 16, mindestens eine Exopeptidase und die nachzuweisende Endopeptidase in einer Lösung; und
bestimme die Stärke der Emission des Fluorophors in der Lösung.

19. Vorrichtung, auf der ein Peptid nach einem der Ansprüche 12 oder 16 immobilisiert ist.

20. Vorrichtung nach dem vorhergehenden Anspruch,
**dadurch gekennzeichnet,**
**dass** das Peptid derart auf der Vorrichtung immobilisiert ist, dass der Fluorophor nach einem Schnitt durch das nachzuweisende Enzym auf der Oberfläche immobilisiert bleibt, während der Quencher nicht mehr auf der Oberfläche immobilisiert ist.

## Claims

1. A peptide with the following properties:
a) the peptide contains an identification sequence of an endopeptidase (enzyme) and can therefore function as substrate for the enzyme, producing a cleavage site between two amino acids at the identification sequence in the peptide;
b) the peptide contains at least one quencher and at least one fluorophore;
c) the quencher is the amino acid tryptophan;
d) the fluorophore is a fluorescent dye;
e) the fluorescent dye can couple covalently to peptides or proteins;
f) the fluorescence of the fluorescent dye can be quenched by tryptophan via photo-induced electron transfer, the tryptophan serving as electron donor;
g) the sequence of the peptide is made up as follows:
h) the quencher is arranged on one side of the cleavage site;
i) the fluorophore is arranged on the other side of the cleavage site;
j) there is sufficient spatial proximity between the quencher and the fluorophore so long as the substrate has not yet been cleaved by the enzyme at the cleavage site between quencher and fluorophore, to ensure the possibility of an at least partial quenching of the emission of the fluorophore;
k) after cleavage of the substrate by the enzyme at the cleavage site between quencher and fluorophore, a definite increase in emission of the fluorophore is possible.

2. The peptide as claimed in the preceding claim, **characterized in that** the fluorescent dye is an oxazine derivative or a rhodamine derivative.

3. The method for the detection of an endopeptidase (enzyme) with the following steps:
mix a peptide as claimed in one of the preceding claims and the enzyme to be detected in a solution; and
determine the intensity of emission of the fluorophore in the solution.

4. A device on which a peptide as claimed in claim 1 or 2 is immobilized.

5. The device as claimed in the preceding claim, **characterized in that** the peptide is immobilized on the device in such a way that after cleavage by the enzyme to be detected, the fluorophore remains immobilized on the surface, whereas the quencher is no longer immobilized on the surface.

6. A peptide with the following properties:
a) the peptide contains at least one group that contains a quencher and a fluorophore on different amino acids;
b) the quencher is the amino acid tryptophan;
c) the fluorophore is a fluorescent dye;
d) the fluorescent dye can couple covalently to peptides or proteins;
e) the fluorescence of the fluorescent dye can be quenched by tryptophan;
f) the sequence of the peptide is made up as follows:
g) there is sufficient spatial proximity between the quencher and the fluorophore so long as the amino acids carrying the quencher and the fluorescent dye have not yet been separated by an exopeptidase (enzyme), to ensure the possibility of an at least partial quenching of the emission of the fluorophore;
l) after separation of the amino acids carrying the quencher and the fluorescent dye by the enzyme, a sufficient spatial distance is ensured between the quencher or any other quencher and the fluorophore, to ensure an at least partial increase in emission of the fluorophore.

7. The peptide as claimed in the preceding claim, **characterized in that** the fluorescent dye is an oxazine derivative or a rhodamine derivative.

8. The peptide as claimed in one of the two preceding claims, **characterized in that**:
the quencher is at least one first fluorescent dye;
the fluorophore is at least one second fluorescent dye;
the at least one first and one second fluorescent dye can couple covalently to peptides or proteins; and
the at least one first and second fluorescent dye can quench each other.

9. The method for the detection of an exopeptidase (enzyme) with the following steps:
mix a peptide as claimed in one of the three preceding claims and the enzyme to be detected in a solution; and
determine the intensity of emission of the fluorophore in the solution.

10. A device on which a peptide as claimed in one of the claims 6 to 8 is immobilized.

11. The device as claimed in the preceding claim, **characterized in that** the peptide is immobilized on the device in such a way that after cleavage by the enzyme to be detected, the fluorophore remains immobilized on the surface, whereas the quencher is no longer immobilized on the surface.

12. A peptide with the following properties:
a) a group that prevents an exopeptidase from digesting the peptide (stop group) is arranged at one end of the peptide;
b) the peptide contains an identification sequence of an endopeptidase (enzyme) and can therefore function as substrate for the enzyme, producing a cleavage site between two amino acids at the identification sequence in the peptide;
c) viewed from the stop group, the peptide contains, in the peptide sequence on the other side of the cleavage site, at least one group that contains a quencher and a fluorophore on different amino acids;
d) the sequence of the peptide is made up as follows:
e) there is sufficient spatial proximity between the quencher and the fluorophore, so long as the amino acids carrying the quencher and the fluorescent dye have not yet been separated by an exopeptidase, to ensure the possibility of an at least partial quenching of the emission of the fluorophore;
f) after separation of the amino acids carrying the quencher and the fluorescent dye by an exopeptidase, a sufficient spatial distance is ensured between the quencher or any other quencher and the fluorophore, to ensure an at least partial increase in emission of the fluorophore.

13. The peptide as claimed in the preceding claim, **characterized in that**:
the quencher is the amino acid tryptophan;
the fluorophore is a fluorescent dye;
the fluorescent dye can couple covalently to peptides or proteins; and
the fluorescence of the fluorescent dye can be quenched by tryptophan.

14. The peptide as claimed in the preceding claim, **characterized in that** the fluorescent dye is an oxazine derivative or a rhodamine derivative.

15. The peptide as claimed in claim 12, **characterized in that**:
the quencher is at least one first fluorescent dye;
the fluorophore is at least one second fluorescent dye;
the at least one first and second fluorescent dye can couple covalently to peptides or proteins; and
the at least one first and second fluorescent dye can quench each other.

16. The peptide as claimed in the preceding claim, **characterized in that** the fluorescent dye is an oxazine derivative or a rhodamine derivative or a cyanine derivative.

17. A kit with a peptide as claimed in one of the five preceding claims and with at least one exopeptidase.

18. A method for the detection of an endopeptidase with the following steps;
mix a peptide as claimed in one of the claims 12 to 16, at least one exopeptidase and the endopeptidase to be detected, in a solution; and
determine the intensity of emission of the fluorophore in the solution.

19. A device on which a peptide as claimed in one of the claims 12 or 16 is immobilized.

20. The device as claimed in the preceding claim, **characterized in that** the peptide is immobilized on the device in such a way that after cleavage by the enzyme to be detected, the fluorophore remains immobilized on the surface, whereas the quencher is no longer immobilized on the surface.

## Revendications

1. Peptide ayant les propriétés suivantes :
a) le peptide contient un site de reconnaissance pour une endopeptidase (enzyme) et peut par conséquent fonctionner comme substrat pour l'enzyme, un site de coupure entre deux aminoacides auprès de la séquence de reconnaissance en résultant dans le peptide ;
b) le peptide contient au moins un extincteur et au moins un fluorophore ;
c) l'extincteur est l'aminoacide tryptophane ;
d) le fluorophore est un colorant fluorescent ;
e) le colorant fluorescent peut être couplé par covalence à des peptides ou des protéines ;
f) la fluorescence du colorant fluorescent peut être éteinte par le tryptophane par transfert photo-induit d'électrons, le tryptophane agissant comme donneur d'électrons ;
g) la séquence du peptide est constituée comme suit :
h) l'extincteur est disposé d'un côté du site de coupure ;
i) le fluorophore est disposé de l'autre côté du site de coupure ;
j) il existe une proximité spatiale suffisante entre extincteur et fluorophore, tant que le substrat n'a pas encore été coupé par l'enzyme au niveau du site de coupure entre extincteur et fluorophore, pour assurer la possibilité d'une extinction au moins partielle de l'émission du fluorophore ;
k) après la coupure du substrat par l'enzyme au niveau du site de coupure entre extincteur et fluorophore une nette augmentation de l'émission du fluorophore est possible.

2. Peptide selon la revendication précédente, **caractérisé en ce que** le colorant fluorescent est un dérivé d'oxazine ou un dérivé de rhodamine.

3. Procédé pour la détection d'une endopeptidase (enzyme), comportant les étapes suivantes :
- mélange d'un peptide selon l'une quelconque des revendications précédentes et de l'enzyme à détecter dans une solution ; et
- détermination de l'intensité de l'émission du fluorophore dans la solution.

4. Dispositif, sur lequel est immobilisé un peptide selon la revendication 1 ou 2.

5. Dispositif selon la revendication précédente, **caractérisé en ce que** le peptide est immobilisé sur le dispositif de manière qu'après une coupure par l'enzyme à détecter le fluorophore reste immobilisé sur la surface, tandis que l'extincteur n'est plus immobilisé sur la surface.

6. Peptide ayant les propriétés suivantes :
a) le peptide contient au moins un groupe qui contient un extincteur et un fluorophore sur des aminoacides différents ;
b) l'extincteur est l'aminoacide tryptophane ;
c) le fluorophore est un colorant fluorescent ;
d) le colorant fluorescent peut être couplé par covalence à des peptides ou des protéines ;
e) la fluorescence du colorant fluorescent peut être éteinte par le tryptophane ;
f) la séquence du peptide est constituée comme suit :
g) il existe une proximité spatiale suffisante entre extincteur et fluorophore, tant que les aminoacides portant l'extincteur ou, respectivement, le colorant fluorescent n'ont pas encore été séparés par une exopeptidase (enzyme), pour assurer la possibilité d'une extinction au moins partielle de l'émission du fluorophore ;
l) après séparation par l'enzyme des aminoacides portant l'extincteur ou, respectivement, le colorant fluorescent, est assurée un éloignement spatial suffisant entre un éventuellement autre extincteur et le fluorophore pour assurer une augmentation au moins partielle de l'émission du fluorophore.

7. Peptide selon la revendication précédente, **caractérisé en ce que** le colorant fluorescent est un dérivé d'oxazine ou un dérivé de rhodamine.

8. Peptide selon l'une quelconque des deux revendications précédentes, **caractérisé**
**en ce que** l'extincteur est au moins un premier colorant fluorescent ;
**en ce que** le fluorophore est au moins un deuxième colorant fluorescent ;
**en ce que** ledit au moins un premier et ledit au moins un deuxième colorant fluorescent sont susceptibles de couplage par covalence à des peptides ou des protéines ; et
**en ce que** ledit au moins un premier et ledit au moins un deuxième colorant fluorescent peuvent s'éteindre mutuellement.

9. Procédé pour la détection d'une exopeptidase (enzyme) comportant les étapes suivantes :
- mélange d'un peptide selon l'une quelconque des trois revendications précédentes et de l'enzyme à détecter dans une solution ; et
- détermination de l'intensité de l'émission du fluorophore dans la solution.

10. Dispositif, sur lequel est immobilisé un peptide selon l'une quelconque des revendications 6 à 8.

11. Dispositif selon la revendication précédente, **caractérisé en ce que** le peptide est immobilisé sur le dispositif de manière qu'après une coupure par l'enzyme à détecter le fluorophore reste immobilisé sur la surface, tandis que l'extincteur n'est plus immobilisé sur la surface.

12. Peptide ayant les propriétés suivantes :
a) à une extrémité du peptide est disposé un groupe qui empêche qu'une exopeptidase digère le peptide (groupe d'arrêt) ;
b) le peptide contient un site de reconnaissance pour une endopeptidase (enzyme) et peut par conséquent fonctionner comme substrat pour l'enzyme, un site de coupure entre deux aminoacides dans la séquence de reconnaissance en résultant dans le peptide ;
c) le peptide contient, vu du groupe d'arrêt dans la séquence du peptide au-delà du site de coupure, au moins un groupe qui contient un extincteur et un fluorophore sur des aminoacides distincts ;
d) la séquence du peptide est constituée comme suit :
e) il existe une proximité spatiale suffisante entre extincteur et fluorophore, tant que les aminoacides portant l'extincteur ou, respectivement, le colorant fluorescent n'ont pas encore été séparés par une exopeptidase, pour assurer la possibilité d'une extinction au moins partielle de l'émission du fluorophore ;
f) après séparation par une exopeptidase des aminoacides portant l'extincteur ou, respectivement, le colorant fluorescent, est assuré un éloignement spatial suffisant entre un éventuellement autre extincteur et le fluorophore pour assurer une augmentation au moins partielle de l'émission du fluorophore.

13. Peptide selon la revendication précédente,
**caractérisé**
**en ce que** l'extincteur est l'aminoacide tryptophane ;
**en ce que** le fluorophore est un colorant fluorescent ;
**en ce que** le colorant fluorescent peut être couplé par covalence à des peptides ou des protéines ; et en ce que la fluorescence du colorant fluorescent peut être éteinte par le tryptophane.

14. Peptide selon la revendication précédente, **caractérisé en ce que** le colorant fluorescent est un dérivé d'oxazine ou un dérivé de rhodamine.

15. Peptide selon la revendication 12, **caractérisé**
**en ce que** l'extincteur est au moins un premier colorant fluorescent ;
**en ce que** le fluorophore est au moins un deuxième colorant fluorescent ;
**en ce que** ledit au moins un premier et ledit au moins un deuxième colorant fluorescent sont susceptibles de couplage par covalence à des peptides ou des protéines ; et
**en ce que** ledit au moins un premier et ledit au moins un deuxième colorant fluorescent peuvent s'éteindre mutuellement.

16. Peptide selon la revendication précédente, **caractérisé en ce que** le colorant fluorescent est un dérivé d'oxazine ou un dérivé de rhodamine ou un dérivé de cyanine.

17. Nécessaire, comportant un peptide selon l'une quelconque des cinq revendications précédentes et comportant au moins une exopeptidase.

18. Procédé pour la détection d'une endopeptidase, comportant les étapes suivantes :
- mélange d'un peptide selon l'une quelconque des revendications 12 à 16, d'au moins une exopeptidase et de l'endopeptidase à détecter dans une solution ; et
- détermination de l'intensité de l'émission du fluorophore dans la solution.

19. Dispositif, sur lequel est immobilisé un peptide selon l'une quelconque des revendications 12 et 16.

20. Dispositif selon la revendication précédente, **caractérisé en ce que** le peptide est immobilisé sur le dispositif de manière qu'après une coupure par l'enzyme à détecter le fluorophore reste immobilisé sur la surface, tandis que l'extincteur n'est plus immobilisé sur la surface.
